# FASCICULE DE BREVET EUROPEEN

(11) **EP 3 174 473 B1**
(45) Date de publication et mention de la délivrance du brevet: **28.11.2018**
(21) Numéro de dépôt: 15745541.1
(22) Date de dépôt: 27.07.2015
(51) Int. Cl.: A61B 17/16

(54) **PORTE-OUTIL CHIRURGICAL UNIVERSEL MULTIVOIE**
UNIVERSELLER MEHRWEGHALTER FÜR CHIRURGISCHE INSTRUMENTE
MULTI-PATH UNIVERSAL SURGICAL TOOL HOLDER

(30) Priorité: 30.07.2014 FR 1457366
(43) Date de publication de la demande: 07.06.2017
(73) Titulaire: Orthoconsult, 76130 Mont-Saint-Aignan (FR)
(72) Inventeur: ADRIAN, Denis, 76130 Mont-Saint-Aignan (FR)
(74) Mandataire: Schmidt, Martin Peter
(86) Numéro de dépôt international: PCT/FR2015/052072
(87) Numéro de publication internationale: WO 2016/016565

(56) Documents cités:
- EP-A1- 2 363 076
- FR-A1- 2 926 208
- FR-A1- 2 961 684
- US-A- 5 443 471
- US-A1- 2010 121 331
- US-B1- 6 205 884

## Description

### Domaine technique de l'invention

L'invention se rattache au domaine des implants orthopédiques et notamment à l'instrumentation qui sert à préparer et à réaliser la pose d'une prothèse et, plus particulièrement, la pièce fémorale d'une prothèse de hanche.

### Etat de la technique

Une prothèse de hanche comporte deux ensembles essentiels : un implant fémoral fixé sur l'os du fémur et un implant cotyloïdien fixé sur l'os iliaque. La coopération de ces deux implants est destinée à rendre à un patient porteur de la prothèse de hanche les mêmes capacités de mouvements et de déplacements que celles permises par une hanche naturelle. Une prothèse totale de hanche comprend de façon classique, d'une part, une partie fémorale constituée d'une tige sur l'extrémité supérieure de laquelle est fixée une tête fémorale et d'autre part une partie cotyloïdienne à fixer dans l'os de bassin comportant par exemple une cupule métallique cotyloïdienne en forme de demi-sphère à l'intérieur de laquelle est logé un insert en matière plastique ou en céramique, dans lequel vient s'articuler la tête fémorale.

Typiquement, pour façonner le fémur avant l'implantation d'une tige fémorale dans une arthroplastie de hanche, i.e. lorsque la tête fémorale usée est remplacée, le praticien doit dans un premier temps préparer la cavité médullaire du fémur qui recevra la tige de la prothèse. Ces opérations de préparation du fut médullaire comprennent principalement des opératoires d'alésage du fut fémoral pour obtenir une première cavité à l'intérieur de l'os, puis de creusement de celle-ci pour lui donner une forme et des dimensions proches de celles de l'implant définitif. L'opération de préparation et de creusement de la cavité alésée se fait généralement à l'aide de râpes fémorales de dimension croissante passées successivement dans la cavité du fémur, les râpes présentant généralement une forme proche de celle de l'implant définitif mais ayant de plus faibles dimensions que ce dernier. Les râpes sont généralement munies de stries ou dents sur leur corps pour pouvoir enlever de la matière osseuse. De telles râpes sont bien connues de l'homme du métier et sont par exemple décrites dans le document FR 2 961 684 A1. Les râpes sont adaptées sur un outil préhenseur nommé « porte râpe » et dont le système de préhension et d'encliquetage dépend des caractéristiques de la râpe et est donc fournie généralement par le fabricant de la prothèse. Une fois la cavité médullaire du fémur préparée, le praticien peut réaliser la pose de la prothèse fémorale dans la cavité.

Une prothèse de hanche peut être implantée dans la cavité médullaire du fémur selon plusieurs approches connues appelées voies d'abord. On peut citer notamment les voies d'abord postérieures (voie d'abord postéro-externe appelée aussi voie de Moore), les voies d'abord antérieures, par exemple la voie d'abord antérieure de Hueter ou sa modification dite de Judet-Letournel qui nécessite l'usage d'une table orthopédique, les voies externes (trans-fessières ou voie de Hardinge par exemple). Il existe aussi la voie antéro-externe de Watson-Jones avec son procédé récemment modifié de Rottinger. Dans les pays nordiques ou anglo-saxons la voie antérieure directe (Direct Anterior Approach ou DAA) est très fréquemment utilisée. Ces voies d'abord sont connues de l'homme du métier et ont été décrites dans des nombreux articles et ouvrages (voir par exemple: D. Kelmanovich et al., « Surgical Approaches to Total Hip Arthroplasty », Journal of the Southern Orthopedic Association 12(2) :90-94, 2003 ; F. Rachbauer et al., « The History of the Anterior Approach to the Hip », Orthop. Clin. N Am 40 (2009) 311-320 ; voir en particulier les articles suivants parus dans les Cahiers d'enseignement de la SOFCOT vol 90, Elsevier 2005 : F. Duparc, « Anatomie chirurgicale de la hanche appliquée à l'arthroplastie prothétique de première intention » (p. 226-230) ; T. Judet et al., « Voie d'abord antérieure et prothèse de hanche de première intention » (p. 237-248) ; J.-Y. Hery et S. Terver, « Voie d'abord de Watson-Jones » p. 249-256) ; J.-L. Tricoire « Voies latérales » (p. 262-268) ; P. Chiron et al. « Voie d'abord postérieure pour prothèse totale de la hanche » (p. 269-283). Les voies d'abord latérales principalement connues sous le nom de voie de Hardinge ont l'inconvénient d'imposer la désinsertion et la réinsertion partielles des muscles petit et moyen fessiers, moteurs et stabilisateurs essentiels de la hanche et pour cette raison sont progressivement délaissées au profit des voies d'abord antérieures et postérieures qui respectent intégralement les petit et moyen fessiers.

La définition même de voie d'abord antérieure ou postérieure est déterminée par rapport à l'éventail des muscles petit et moyen fessiers qu'elles respectent intégralement alors que les voies externes les traversent. Les deux voies d'abord principalement utilisées (antérieure et postérieure) présentent chacune des avantages et des inconvénients. De plus, l'utilisation par le praticien d'une voie par rapport à une autre est déterminée aussi bien par un simple choix personnel que par les comorbidités et demandes fonctionnelles du patient. De même, l'installation du patient durant l'intervention, en décubitus dorsal ou en décubitus latéral, est une variable qui dépend des habitudes et des choix du chirurgien, notamment pour les voies d'abord antérieures. La voie d'abord antérieure peut être préférée pour des interventions sur des jeunes patients car cette voie respecte intégralement la musculature, elle est donc très peu traumatisante et autorise une revalidation rapide. Elle est techniquement exigeante pour le praticien. Pour les patients plus âgés, la voie d'abord postérieure peut être préférée car elle est rapide et facile d'exécution, entrainant un très faible sacrifice tendineux musculaire et le travail du fût fémoral est plus aisé que pour les voies antérieures. A ce jour, il n'existe pas d'étude démontrant scientifiquement une supériorité d'une voie d'abord par rapport à une autre. Les voies d'abord antérieures et postérieures ont été décrites et modifiées par de nombreux praticiens comme décrit plus haut.

Actuellement selon la voie d'abord choisie par le praticien, le nombre et le type d'outils chirurgicaux et notamment de porte râpes nécessaires pour réaliser la pose de la tige fémorale doivent être différents. De plus, l'évolution des interventions vers une chirurgie dite mini invasive, i.e. réduisant au maximum la taille de l'incision cutanée, a augmenté de manière significative le nombre d'instruments pour minimiser les lésions éventuelles des parties molles (muscles, tendons, ligaments) de l'articulation coxo-fémorale, tout en contournant les reliefs et saillies osseuses pouvant faire obstacle au travail du fut fémoral (massif trochantérien du fémur, aile et crête de l'os iliaque). Le travail de préparation du fut fémoral doit permettre la pose de l'implant de la façon la plus satisfaisante possible, i.e. dans l'axe de la diaphyse fémorale. On appelle Valgus une déviation axiale ou une force s'exerçant vers le dehors de l'axe du fut fémoral et Varus une force ou une déviation axiale en dedans de la diaphyse fémorale, l'axe de référence étant l'axe de la diaphyse fémorale. Une pose axiale parfaite est l'idéal, une pose en valgus modéré est tolérée, mais une implantation en varus est à éviter sur le plan mécanique et anatomique. La tendance naturelle du conflit avec les parties molles ou les reliefs osseux (trochantérien notamment) est de favoriser une implantation défectueuse en varus de façon encore plus prononcée dans les techniques mini invasives.

Actuellement pour une implantation d'une prothèse totale de la hanche pour laquelle la voie antérieure est sélectionnée, le praticien aura besoin d'un porte-râpe adapté à la voie d'abord utilisée qui sera différent selon le type de voie antérieure et l'installation en décubitus dorsal ou décubitus latéral. De même le porte râpe sera encore différent si le praticien a sélectionné une voie postérieure. Ainsi, l'ensemble de ces techniques impose aux centres d'opération de disposer d'une gamme importante d'outils chirurgicaux adaptés à la technique, à la voie d'abord utilisé et à la pratique du chirurgien, en moyenne de 5 à 7 instruments par gamme d'implants fémoraux de prothèse de hanche pour répondre à toutes les situations. D'autre part, ces instruments doivent pouvoir être désinfectés facilement sans cavité inaccessible pour les opérateurs habilités au nettoyage. Pour l'instant, les tentatives de normalisation et de standardisation des porte-râpes fémoraux sont restées infructueuses.

US 2010/0121331 divulgue un porte-outil, dont la masse de frappe comprend des méplats qui créent des discontinuités dans la zone de frappe d'insertion. Cette masse de frappe possède cependant une semelle inférieure continûment plane, de sorte que l'impaction et l'extraction du porte-outil sont réalisées uniquement de façon axiale.

US 6,205,884 décrit un porte outil, dont la masse de frappe comporte deux zones. Sur certaines figures de ce document, ces deux zones sont très légèrement décalées mutuellement, selon un angle très faible. En revanche, d'autres figures montrent que ces deux zones s'étendent dans le prolongement l'une de l'autre. En tout état de cause, ce document ne délivre aucun enseignement technique clair à l'homme du métier, en ce qui concerne les directions d'impaction et d'extraction.

Enfin US 5,443,471 a pour objet un porte-outil pourvu d'une plaque pouvant assurer une fonction, soit de frappe, soit de préhension. A cet effet, la zone principale de la plaque, s'étendant horizontalement en service, est adaptée pour cette fonction de frappe. Par ailleurs, la zone secondaire de cette plaque, s'étendant obliquement en service, est uniquement adaptée pour la fonction de préhension, mais en revanche pas pour la fonction de frappe.

Le problème que se propose de résoudre l'invention, consiste à concevoir des outils chirurgicaux (porte râpes) compatibles avec toutes les voies d'abord actuellement décrites, préférentiellement réutilisables et compactes pour être utilisé selon les techniques opératoires que le praticien ait sélectionné et permettant une fixation stable, solide et durable de l'implant, tout en réduisant le nombre d'outils aptes à travailler les cavités médullaires correspondant à une gamme d'implant (en moyenne une paire d'instruments symétriques et réversibles permettant de faire face à toutes les situations).

Dans le cadre de la présente invention, la demanderesse a mis au point un ensemble répondant aux inconvénients cités précédemment, en concevant un ensemble de porte-râpes, polyvalents et réversibles, compatibles avec toutes les voies d'abord antérieures et postérieures connues du praticien, aussi bien en décubitus dorsal que latéral, et pouvant, selon les souhaits du fabriquant de prothèse, être compatibles avec tous les modes de fixation de la râpe sur ledit ensemble de porte-râpe (i.e. de l'outil sur le manche porte-outil) et à permettre la préparation du fut fémoral et l'implantation de l'implant de la façon la plus axiale et parfaite possible dans toutes les situations.

### Objets de l'invention

Un premier objet selon l'invention concerne un porte-outil chirurgical pour fixer un outil-chirurgical comportant : un corps de manche comprenant une partie proximale et une partie distale délimitée par un décrochement ; une masse de frappe située à l'extrémité de la partie proximale dudit corps de manche; un moyen de fixation d'un outil chirurgical situé à l'extrémité distale du corps de manche, ledit porte-outil étant caractérisé en ce que ladite masse de frappe, avantageusement en forme de virgule ou d'accent circonflexe, comprend une partie supérieure et une partie inférieure, ladite partie supérieure comprenant une zone de frappe d'insertion dans l'axe du fémur et de la râpe, légèrement courbe au besoin, et s'étendant selon un premier axe longitudinal (B-B') ; et une zone de frappe d'insertion dans l'axe de la partie proximale de l'instrument de forme sensiblement plate s'étendant selon un deuxième axe longitudinal (A-A'), l'angle α formé par l'intersection des premier et deuxième axes longitudinaux (B-B' ; A-A') étant compris entre 20° et 50°, de préférence entre 25° et 45° ; une valeur d'environ 35° est la plus préférée. Dans un mode de réalisation, la partie proximale du corps de manche s'étend de manière longitudinale selon un troisième axe (C-C'), la partie distale dudit corps de manche s'entend de manière longitudinale selon un axe (D-D'), et en ce que l'angle β formé par l'intersection des axes (C-C' ; D-D') est compris entre 20 et 45°, de préférence entre 25 et 40°, et encore plus préférentiellement autour entre 32° et 38° ; une valeur d'environ 35° est la plus préférée.

Dans un autre mode de réalisation pouvant être combiné avec le précédent, l'outil chirurgical apte à coopérer avec ledit porte-outil s'étend de manière longitudinal selon un cinquième axe (E-E'), l'angle γ formé par l'intersection entre le deuxième axe longitudinal (B-B') et ledit cinquième axe (E-E') étant compris entre 87° et 93°, de préférence autour de 90°.

En tous les cas, ledit porte-outil comprend avantageusement un organe de manoeuvre comprenant un actionneur. L'organe de commande permet de commander ledit moyen de fixation d'un outil chirurgical. Dans un mode de réalisation ledit actionneur agit sur une tige logée dans le corps de manche qui, elle, agit sur ledit moyen de fixation. L'actionneur est de préférence logé dans une lumière longitudinale du corps de manche.

Ledit actionneur de l'organe de commande est apte à être déplacé longitudinalement ou latéralement (comme un levier) pour verrouiller ou déverrouiller le moyen de fixation.

La tige guide de l'organe de commande est en appui sur un ressort pour verrouiller ou déverrouiller les moyens de fixation. La tige guide présente un décrochement de même rayon de courbure que le décrochement dudit corps de manche.

Les moyens de fixation dudit porte-outil se présentent sous la forme d'au moins un loquet de fixation.

Ledit porte-outil peut comprendre en outre un orifice d'orientation angulaire situé sur la partie proximale du corps de manche pour l'introduction d'une tige cylindrique afin d'orienter angulairement un outil chirurgical, tel qu'une râpe ou une tige fémorale.

De façon avantageuse le moyen de fixation définit un cinquième axe longitudinal confondu avec l'axe principal de l'outil chirurgical, quand cet outil est fixé sur le porte-outil, ledit cinquième axe formant, avec l'axe principal du corps de manche, le même angle (α) que celui formé par l'intersection des premier et deuxième axes longitudinaux.

De façon avantageuse, la partie inférieure délimite avec la partie proximale dudit corps de manche, en regard de la zone de frappe d'insertion dans l'axe du manche du porte râpe, un espace de passage pour un outil, en vue de l'extraction du porte-outil selon l'axe du manche du porte râpe.

Ledit porte-outil selon l'invention est apte à venir en prise avec au moins une râpe, telle qu'une râpe de préparation ou de finition, et/ou avec au moins une tige fémorale.

Un autre objet de l'invention est un ensemble constitué par un porte-outil tel que décrit ci-dessus et un outil chirurgical, tel qu'une râpe ou une tige fémorale, ledit outil chirurgical étant disposé de manière à ce que sont axe longitudinal s'étende selon un cinquième axe (E-E'), l'angle γ formé par l'intersection entre ledit deuxième axe longitudinal (B-B') et ledit cinquième axe (E-E') étant compris entre 87° et 93°, de préférence autour de 90°.

En particulier, ledit cinquième axe forme, avec l'axe principal du corps de manche, le même angle que celui formé par l'intersection des premier et deuxième axes longitudinaux.

Encore un autre objet concerne un set d'outils chirurgicaux comprenant un porte-outil chirurgical selon l'invention, et au moins une râpe de finition et/ou au moins une râpe de préparation et/ou au moins une tige fémorale de prothèse de hanche aptes à coopérer avec ledit porte-outil chirurgical par l'intermédiaire des moyens de fixation dudit porte-outil.

Encore un autre objet concerne un set d'outils chirurgicaux réversible, bilatéral et symétrique, comprenant des porte-râpes et/ou porte implants pour la préparation et la mise en place d'un implant fémoral de prothèse de hanche, permettant de réduire jusqu'à seulement une paire le nombre de porte râpes et/ou porte implants nécessaires pour la préparation et la mise en place d'un implant fémoral de prothèse de hanche, quelques soient la technique et l'installation du patient ; cela est lié aux différents compromis angulaires du manche et de la platine d'insertion/extraction de l'invention décrite ci-dessous.

### *Description des figures

Les figures 1 à 8 illustrent des modes de réalisation de l'invention. Le porte outil est utilisé à gauche pour les voies antérieures et à droite pour les voies postérieures ; on décrit donc une paire en miroir, la figure étant inversée pour les voies antérieures à droite et postérieures à gauche.
La figure 1 est une vue d'un porte-outil chirurgical selon l'invention coopérant avec une tige fémorale. Sur cette figure, la flèche P₁ représente le sens d'impaction pour une voie postérieure gauche, la flèche A₁ représente le sens d'impaction pour une voie antérieure droite. La flèche P₂ représente le sens pour une voie antérieure droite, la flèche A₂ représente le sens d'extraction pour une voie antérieure droite.
La figure 2 est une frontale d'un porte-outil selon une première variante de réalisation de l'invention. Dans ce mode de réalisation, le porte-outil coopère avec une tige fémorale.
La figure 3 est une vue en perspective du porte-outil coopérant avec une tige fémorale selon la première variante de réalisation de l'invention.
La figure 4 est une vue arrière du porte-outil coopérant avec une tige fémorale modulaire selon la première variante de réalisation de l'invention.
La figure 5 est une frontale d'un porte-outil selon une deuxième variante de réalisation de l'invention. Dans ce mode de réalisation, le porte-outil coopère avec une râpe.
La figure 6 est une vue en perspective du porte-outil coopérant avec une râpe selon la deuxième variante de réalisation de l'invention.
La figure 7 est une vue arrière du porte-outil coopérant avec la râpe selon la deuxième variante de réalisation de l'invention.
La figure 8 est une vue frontale d'un porte-outil coopérant avec une tige fémorale modulaire ladite tige fémorale étant insérée dans le fût fémoral d'un patient, soit par la voie antérieure (ici voie antérieure gauche) soit par la voie d'abord postérieure (ici voie postérieure droite).
La figure 9 est une vue latérale, illustrant le porte-outil conforme à l'invention mis en oeuvre dans une opération par voie antérieure de jambe gauche, selon la technique dite de Judet-Letournel
La figure 10 est un agrandissement de la figure 9
La figure 11 est une vue de dessus du bassin, lors de l'opération des figures 9 et 10
La figure 12 est une vue de dessus, illustrant le porte-outil conforme à l'invention mis en oeuvre dans une opération par voie antérieure de jambe gauche, en position de décubitus latéral, selon la technique dite de Watson Jones / Rottinger
La figure 13 est une vue de dessus du bassin, lors de l'opération de la figure 12 ; et
La figure 14 est une vue de dessus, illustrant le porte-outil conforme à l'invention mis en oeuvre dans une opération par voie postérieure de jambe droite, en position de décubitus latéral.

### Liste des repères :

| | |
|---|---|
| Corps de manche | **1** |
| Axe du corps de manche | **A1** |
| Masse de frappe | **2** |
| Organe de commande | **3** |
| Actionneur longitudinal | **3a** |
| Tige guide | **3b** |
| Décrochement | **3c** |
| Trou d'orientation angulaire | **5** |
| Partie proximale du corps de manche | **6** |
| Partie distale du corps de manche | **7** |
| Décrochement | **8** |
| Moyen de fixation (loquets de fixation) | **9** |
| Porte-outil chirurgical | **10** |
| Partie supérieure (zone d'impaction) | **21** |
| Zone de frappe d'insertion axiale fémorale | **22** |
| Zone de frappe d'insertion dans l'axe du porte râpe (valgus) | **23** |
| Partie inférieure (zone d'extraction) | **24** |
| Zone d'extraction axiale fémorale | **25** |
| Zone d'extraction axe porte râpe (valgus) | **26** |
| Espace de passage d'un outil | **27** |
| Tige fémorale | **30** |
| Tige fémorale (Partie proximale) | **31** |
| Tige fémorale (Partie distale) | **32** |
| Râpe | **40** |
| Râpe (Partie proximale) | **41** |
| Râpe (Partie distale) | **42** |
| Fémur | **50** |
| Fût fémoral | **51** |
| Trochanter | **52** |
| Crête iliaque antérieure | **53** |
| Crête iliaque | **54** |
| Cotyle | **55** |
| Cuisse | **56** |
| Jambe | **57** |
| Genou | **58** |
| Pied | **59** |

### Description détaillée de l'invention

### Définitions

Dans la présente demande, les termes "antérieur" et "postérieur" s'entendent dans leur sens anatomique, c'est-à-dire par rapport à un fémur du squelette humain. Les termes valgus (dehors) et varus (dedans) s'entendent comme des forces ou déviations axiales par rapport à l'axe frontal de la diaphyse fémorale

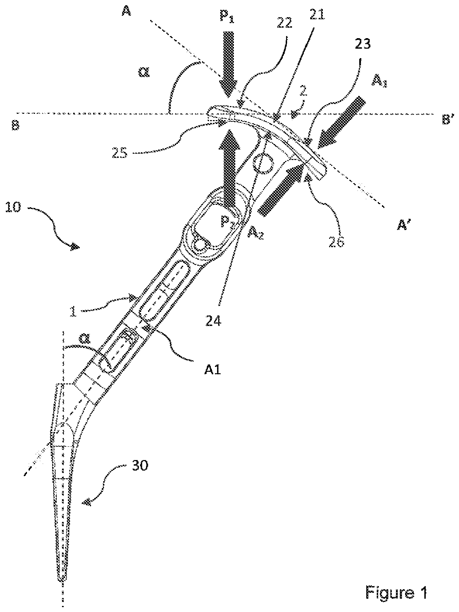

On définit l'angle α comme représentant l'angle compris entre l'axe de diaphyse fémorale et le plan axial du porte-outil selon l'invention. L'angle α se retrouve dans l'angulation de la masse de frappe. Cela est illustré sur les figures 1, 2, 5 et 8.

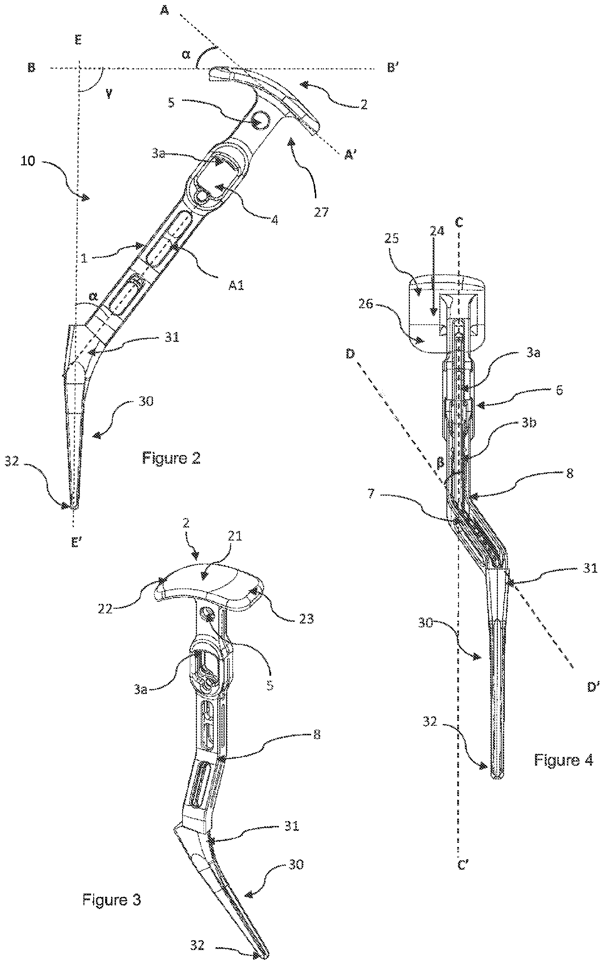

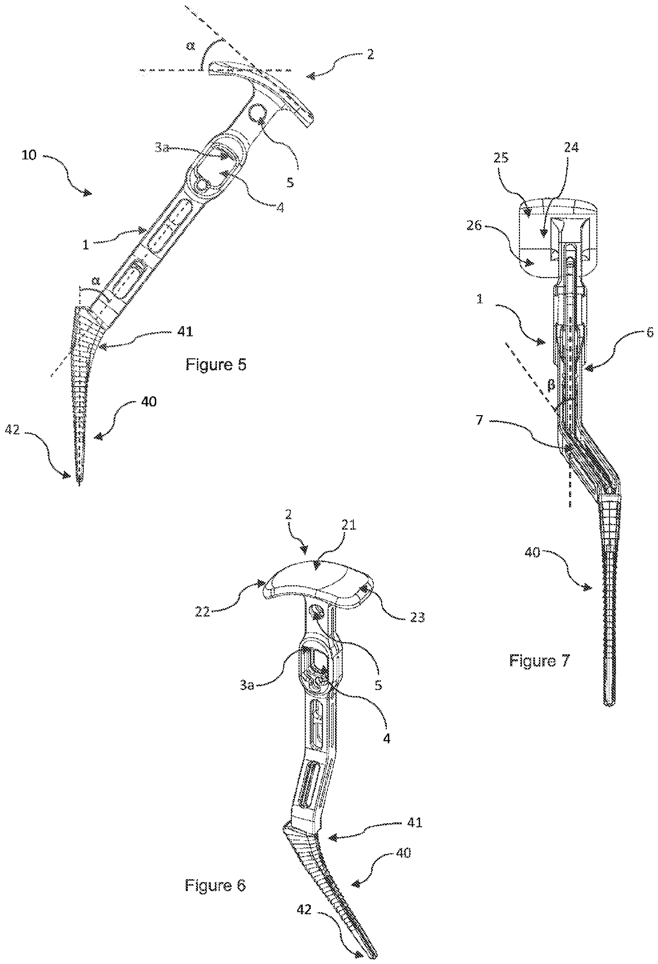

On définit l'angle β comme correspondant à l'angle du double décrochement du porte-outil dans le plan frontal, ce décrochement dans le plan frontal permettant le travail du fémur tout en contournant et en évitant les parties molles musculaires de la cuisse et des reliefs osseux (principalement la saillie de l'aile iliaque). Cela est illustré sur les figures 4 et 7.

### 1. Description

Selon l'invention, pour résoudre ce problème, il a été conçu et mis au point un porte-outil chirurgical pour préparer et poser un implant fémoral selon l'une quelconque des voies d'abord postérieures ou antérieures.

Dans ce qui suit, les faces postérieure et antérieure sont définies pour une tige fémorale de prothèse de hanche gauche (telle que visible sur les figures). Dans le cadre de la présente invention, de multiples études anatomiques et la pratique chirurgicale de l'inventeur ont permis de définir des compromis angulaires qui autorisent la polyvalence et la réversibilité des porte-outils. Par exemple, un porte-outil chirurgical selon l'invention muni d'une râpe pour la hanche droite par voie antérieure se retrouve ainsi idéalement configuré pour une hanche gauche par voie postérieure. Dans les figures 1 à 8, seul le porte-outil permettant une intervention par voie antérieure gauche ou par voie postérieure droite est représenté. L'invention couvre bien évidemment un porte-outil pour une intervention par voie postérieure gauche ou par voir antérieure droite.

Le porte-outil chirurgical **10** selon l'invention est apte à coopérer avec au moins une râpe **40** pour la préparation de la pose de l'implant fémoral, et avec au moins une tige fémorale **30** en vue de son implantation dans le fût fémoral **51** du patient.

Plus particulièrement, et tel que mieux illustré en figure 1, le porte-outil chirurgical **10** comporte :
- un corps de manche **1** comprenant une partie proximale **6** et une partie distale **7** ;
- un actionneur longitudinal **3a** logé dans une lumière longitudinale **4** du corps de manche **1** assujetti à un organe de commande **3** ;
- un moyen de fixation **9** d'au moins un outil chirurgical, en particulier d'au moins une râpe **40** et/ou d'au moins un implant chirurgical, tel qu'une tige fémorale **30** ; et
- une masse de frappe **2** située à l'extrémité proximale **6** du corps de manche **1**.

Le corps de manche **1** comprend une partie proximale **6** s'étendant de manière longitudinale de la masse de frappe **2** à un décrochement **8**, et une seconde partie distale **7** s'étendant du décrochement **8** au moyen de fixation **9** d'au moins une râpe et/ou d'au moins une tige fémorale.

Plus particulièrement, la partie proximale **6** du corps de manche **1** s'étend de manière longitudinale selon un axe C-C', et la partie distale **7** dudit corps de manche **1** s'entend de manière longitudinale selon un axe D-D'. L'intersection des axes C-C' et D-D' forme un angle β, pris entre l'axe longitudinal de la partie proximale **6** du corps de manche 1 et l'axe longitudinal de l'outil chirurgical une fois ce dernier en prise sur le porte-outil, et tel que mieux représenté aux figures 4 et 7. L'angle β, qui est illustré sur la figure 4 en référence à une vue de derrière de la zone de frappe, est avantageusement compris entre 20 et 45°, de préférence entre 25 et 40°, et encore plus préférentiellement autour de 35°. La forme particulière du corps de manche **1** du porte-outil **10** de la présente invention permet au praticien de travailler avec une liberté de mouvement améliorée lors de la préparation du fût fémoral ou de l'implantation de la tige fémorale, et cela aussi bien pour les voies d'abord antérieures que postérieures.

L'organe de commande **3** comprend un actionneur longitudinal **3a** logé dans une lumière longitudinale **4** formée dans l'épaisseur du corps de manche **1**, permettant son nettoyage et sa désinfection rapide en vue d'une réutilisation. L'organe de commande **3** comprend par ailleurs une tige guide **3b** apte à être déplacée longitudinalement pour verrouiller ou déverrouiller le moyens de fixation **9** disposé à l'extrémité de la partie distale **7** distale du corps de manche **1**. Ce moyen de fixation **9**, de type connu en soi, définit un cinquième axe longitudinal E-E', qui correspond à l'axe principal de l'outil chirurgical une fois fixé sur le porte-outil.

Selon un autre aspect particulier, le porte-outil **10** selon l'invention présente une masse de frappe **2** comportant :
- une partie supérieure **21**, appelée aussi ici zone d'impaction **21** ; et
- une partie inférieure **24**, appelée aussi zone d'extraction **24**.

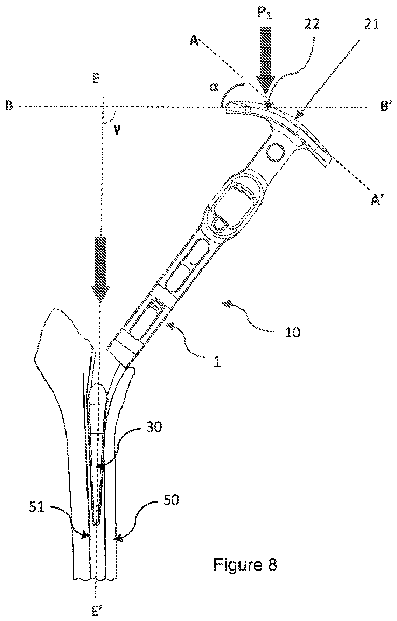

La partie supérieure **21** de la masse de frappe comprend deux zones distinctes, une zone de frappe d'insertion dans l'axe du manche du porte râpe **22** (force exercée en valgus dans le cadre du porte-outil illustré aux figures 1 à 8) et une zone de frappe d'insertion axiale fémorale **23** (dans le cadre du porte-outil tel qu'illustré aux figures 1 à 8). De cette manière, le praticien peut, selon la voie d'abord choisie et les contraintes ou forces à exercer durant la préparation fémorale utiliser le même porte-outil chirurgical selon l'invention. En effet :
- le praticien qui aura choisi la voie d'abord postérieure droite peut utiliser, pour insérer aussi bien une tige fémorale ou une râpe, la zone de frappe d'insertion axiale fémorale par voie postérieure **22** de la partie supérieure **21** de la masse de frappe **2** en exerçant une pression sur ladite zone **22** dans la direction indiquée par la flèche P₁ telle que représentée en figures 1 et 8. La pression exercée s'effectue perpendiculairement à l'axe B-B' et parallèlement à l'axe E-E' dans le sens indiqué par la flèche P₁, i.e. dans l'axe longitudinal du fût fémoral **51**, tel que représenté en figure 8. Cela suppose que l'axe longitudinal de l'outil chirurgical (râpe ou tige fémorale) coïncide approximativement avec l'axe E-E'.
- S'il souhaite appliquer une force en valgus le praticien utilisera la zone 23 de la platine de frappe 21 pour exercer une force dans l'axe du manche du porte râpe dans la direction de la flèche A1 de la figure No1
- le praticien qui aura choisi la voie d'abord antérieure gauche pourra faire de même avec le même instrument

De manière similaire, lors de l'extraction d'une râpe ou d'une tige fémorale, le praticien qui aura choisi la voie d'abord postérieure droite ou la voie antérieure gauche doit utiliser la zone d'extraction axiale fémorale **25** de la partie inférieure **24** de la masse de frappe **2** en exerçant une pression sur ladite zone **25** dans la direction indiqué par la flèche P₂ telle que représentée en figure 1. Cela suppose que l'axe longitudinal de l'outil chirurgical (râpe ou tige fémorale) coïncide approximativement avec l'axe du fût fémoral **51**. Alternativement, le praticien qui aura choisi la voie d'appliquer des forces en valgus ou dans l'axe du porte râpe utilisera la partie **26** de la partie inférieure **24** de la masse de frappe **2** en exerçant une pression sur ladite zone **26** dans la direction indiqué par la flèche A₂ telle que représentée en figure 1.
Lorsque le praticien pratiquera une voie antérieure gauche en décubitus dorsal il utilisera préférentiellement pour l'implantation la partie **23** du corps de frappe **22** (force A1) et pour l'extraction la partie **26** du corps de frappe **22** (force A2). On note, notamment en référence à la figure 2, que la partie inférieure 24 du corps de frappe 22 délimite, avec la partie proximale 6, un espace affecté de la référence 27. Cet espace 27, qui s'étend en regard de la zone de frappe d'insertion dans l'axe du manche du porte râpe, présente des dimensions adaptées pour le passage d'un outil approprié, en vue de cette extraction du porte-outil selon l'axe du manche du porte râpe, c'est à dire selon la flèche A2.

Selon un aspect essentiel du porte-outil **10** selon l'invention, l'angle α, formé par l'axe longitudinal A-A' dans lequel est contenu le plan longitudinal de la zone de frappe d'insertion par voie postérieure **22** et l'axe longitudinal B-B' dans lequel est contenu le plan longitudinal de la zone de frappe d'insertion par voie antérieure **23** (cf. figure 1), est compris entre 20° et 50°, de préférence entre 25° et 45°; une valeur d'environ 35° est la plus préférée. Cet angle α est défini en référence à une vue de face de la zone de frappe, comme sur les figures 1, 2, 5 et 8. Selon cette même vue de face, l'axe E-E' défini ci-dessus forme le même angle α avec l'axe principal A1 du corps de manche, comme cela est notamment visible sur la figure 2.

Selon un autre aspect particulier du porte-outil **10** selon l'invention, les axes longitudinaux B-B' et E-E' forment un angle γ sensiblement égal à 90° (cf. figure 2). En pratique, on peut dévier de quelques degrés de cette valeur idéale, mais une telle déviation risque de dégrader la précision du positionnement de la tige fémorale dans le fût fémoral du patient. Ainsi, lors de l'insertion d'une tige fémorale ou d'une râpe dans le fût fémoral **51** d'un patient par la voie d'abord postérieure droite ou voie antérieure gauche, la pression exercée sur la zone de frappe d'insertion **22** de la masse de frappe **2** dans la direction indiquée par la flèche P₁ permet une insertion de ladite tige fémoral ou de ladite râpe dans l'axe longitudinal fémoral du patient sans risque de blocage.

Ainsi, le porte-outil **10** selon l'invention pouvant être indifféremment utilisé en tant qu'outil d'impaction d'une râpe d'un implant (tige fémorale modulaire par exemple) qu'outil d'extraction (d'une râpe ou d'une tige fémorale), et aussi bien en utilisant les voies d'abord antérieures ou postérieures.

L'instrument chirurgical **10** selon l'invention est apte à coopérer avec au moins une râpe **30**, telle que mieux représentées aux figures 5 à 7. De préférence, la râpe **30** comporte un corps allongé recouvert, sensiblement sur toute sa longueur, de reliefs permettant d'éroder et/ou de compacter l'os fémoral. Plus spécifiquement, le corps de râpe comprend une partie proximale **41** destinée à travailler la zone métaphysaire du fémur, et une partie distale **42** destinée à travailler la zone diaphysaire du fémur, la section transversale dudit corps allant en se rétrécissant en direction de la partie distale **42**. La râpe **30** peut aussi bien être une râpe de préparation ou une râpe de finition.

L'instrument chirurgical **10** selon l'invention est également apte à coopérer avec au moins une tige fémorale **40**, telle que représentée aux figures 2 à 4. En particulier, les tiges fémorales divulguées dans le document WO 2010 / 031 922 peuvent convenir.

L'invention présente de nombreux avantages car elle permet d'une part l'introduction de la râpe et le passage de la râpe et du porte-râpe par une incision mini-invasive tout en respectant les divers reliefs osseux et les parties molles environnantes. D'autre part, du fait de la forme particulière de la masse de frappe proximale, le porte-outil selon l'invention permet le travail, l'impaction et l'extraction de la râpe dans l'axe du fémur. Le défaut général de pose des prothèses est d'être en varus, qu'aggravent dans la chirurgie mini-invasive, les conflits avec les parties molles muscles et tendons et le massif du grand trochanter. Une frappe en valgus est parfois nécessaire, utile et tout à fait acceptable, l'autre angulation de la masse de frappe permet l'extraction ou l'impaction de cette façon selon l'angle Alpha du porte manche.

Par ailleurs, le porte-outil selon l'invention permet, dans des courbures d'instruments dédiées à la voie d'abord antérieure ou postérieur, de proposer au praticien un porte-implant et porte-râpe d'utilisation universelle et ceci dans un volume réduit avec une solution technique de verrouillage et de verrouillage des outils chirurgicaux (implant, en particulier tige fémorale, et râpe) tout en permettant une aisance de nettoyage de l'instrument avant stérilisation pour sa réutilisation. Enfin, les différents compromis angulaires permettent l'utilisation d'une paire de porte-outils (droit et gauche) qui sont utilisables de façon réversible et permettent ainsi d'être utilisés dans toutes les voies d'abord. Par exemple, un porte-outil chirurgical selon l'invention muni d'une râpe pour la hanche droite par voie antérieure se retrouve ainsi idéalement configuré pour une hanche gauche par voie postérieure. Alternativement, un porte-outil chirurgical selon l'invention muni d'une râpe pour la hanche gauche par voie antérieure se retrouve ainsi idéalement configuré pour une hanche droite par voie postérieure (voir l'exemple illustré dans le présent document). On rappellera que, par comparaison, il est nécessaire de prévoir au minimum trois paires d'instruments, dans l'art antérieur.

Comme cela ressort de la description ci-dessus, on retrouve avantageusement un même angle remarquable, référencé (α) sur les figures, à deux endroits distincts de la prothèse. Ainsi l'angle entre l'axe principal de l'outil chirurgical, quand cet outil est fixé sur le porte-outil, et l'axe principal du corps de manche, est avantageusement identique à l'angle formé par l'intersection des deux axes longitudinaux des zones de frappe. La reprise de cet angle permet de compenser la désaxation du manche du porte-outil par rapport à l'axe du fémur et, par conséquent, de travailler aussi bien à l'impaction qu'à l'extraction dans les deux conditions idéales, à savoir dans l'axe et en valgus. La reprise de cet angle n'est pas traitée dans l'art antérieur, en particulier dans les documents US 2010/0121331, US 6,205,884 et US 5,443,471 décrits ci-dessus.

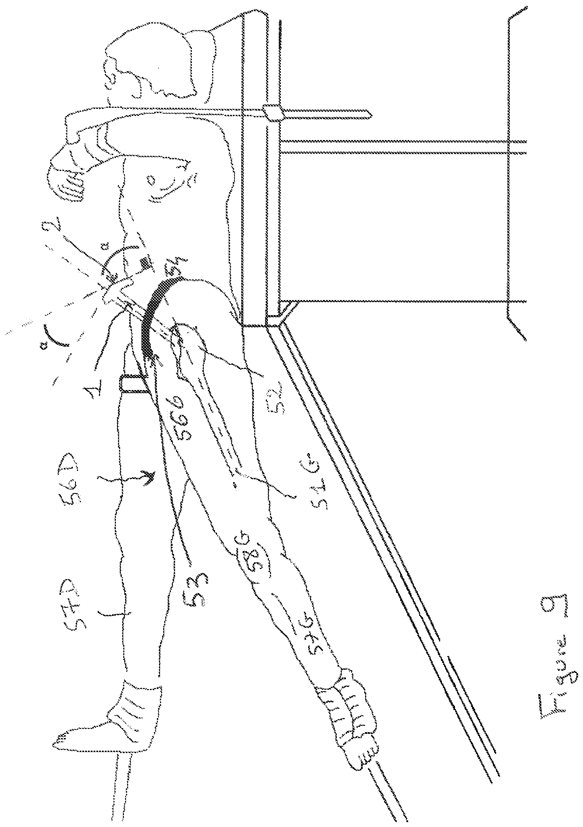

Les figures 9 à 14 illustrent la polyvalence et la réversibilité du porte-outil conforme à l'invention.

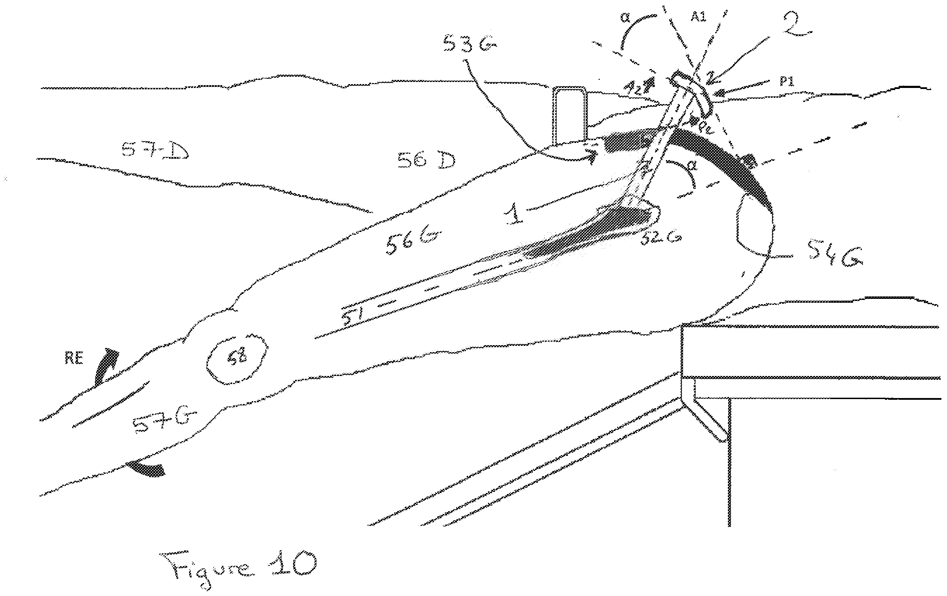

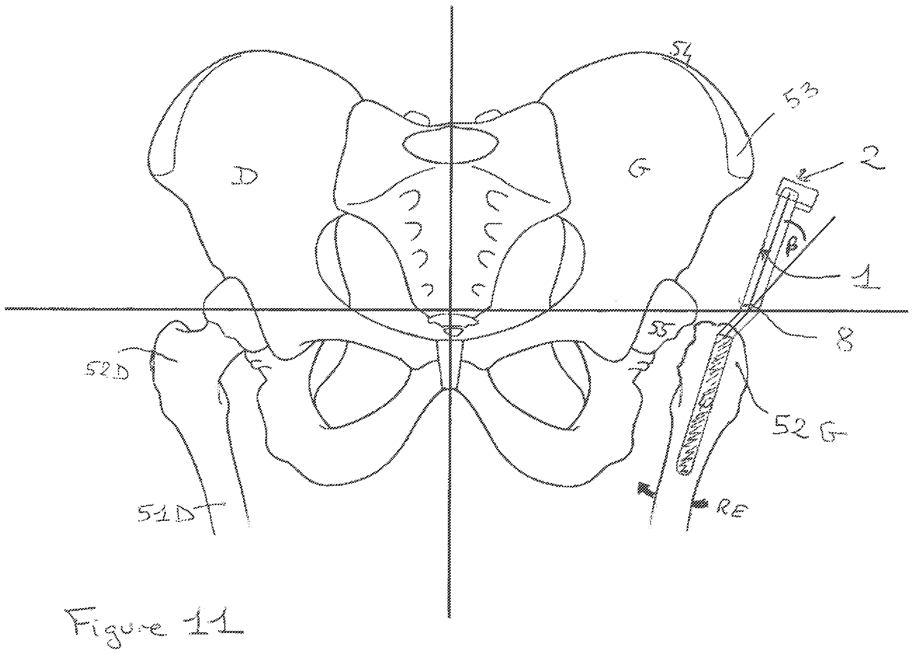

Sur les figures 9 et 10, ce porte-outil est utilisé dans une opération par voie antérieure de jambe gauche, selon la technique dite de Judet-Letournel. La figure 11 montre le bassin du patient, ainsi que le porte-outil, lors de cette opération.

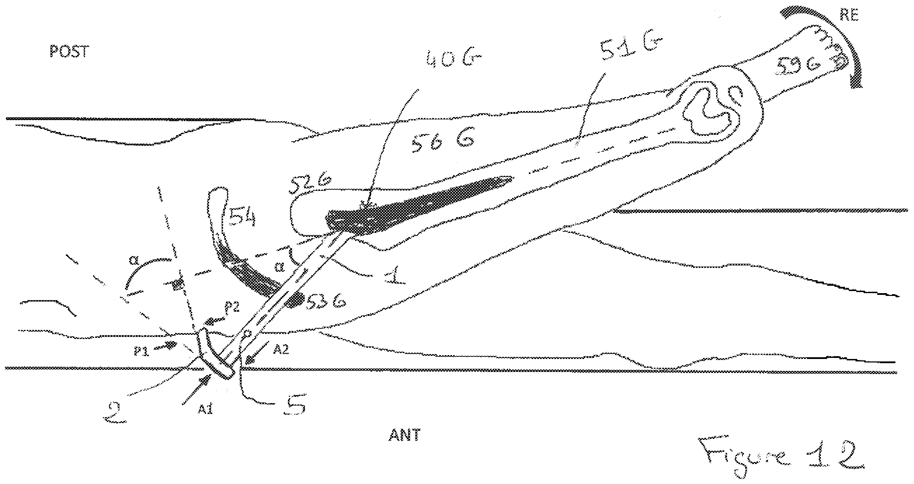

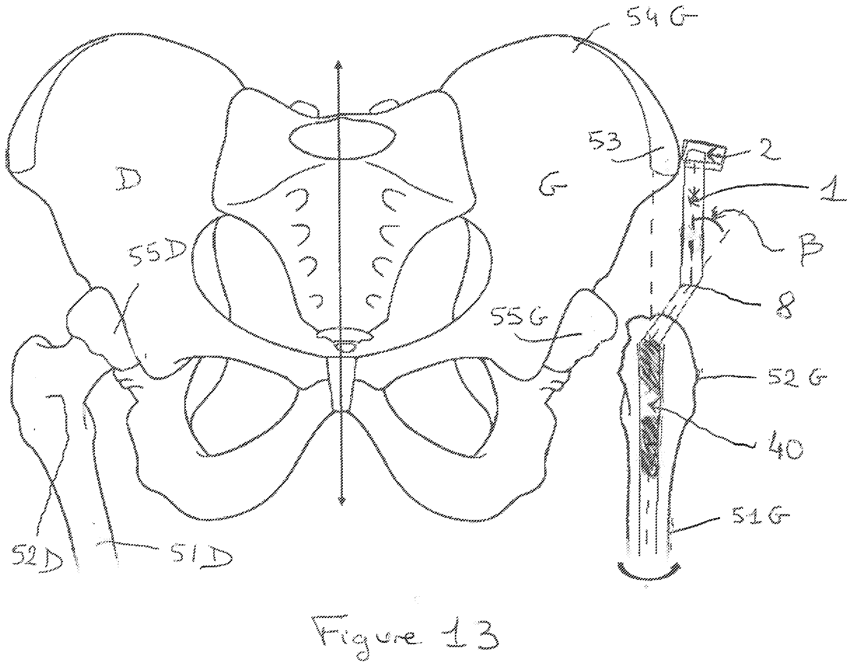

Les figures 12 et 13 illustrent le même porte-outil, conforme à l'invention, utilisé dans une opération par voie antérieure de jambe gauche, selon la technique dite de Watson Jones / Rottinger. Sur la figure 12, on retrouve le patient en position de décubitus latéral,

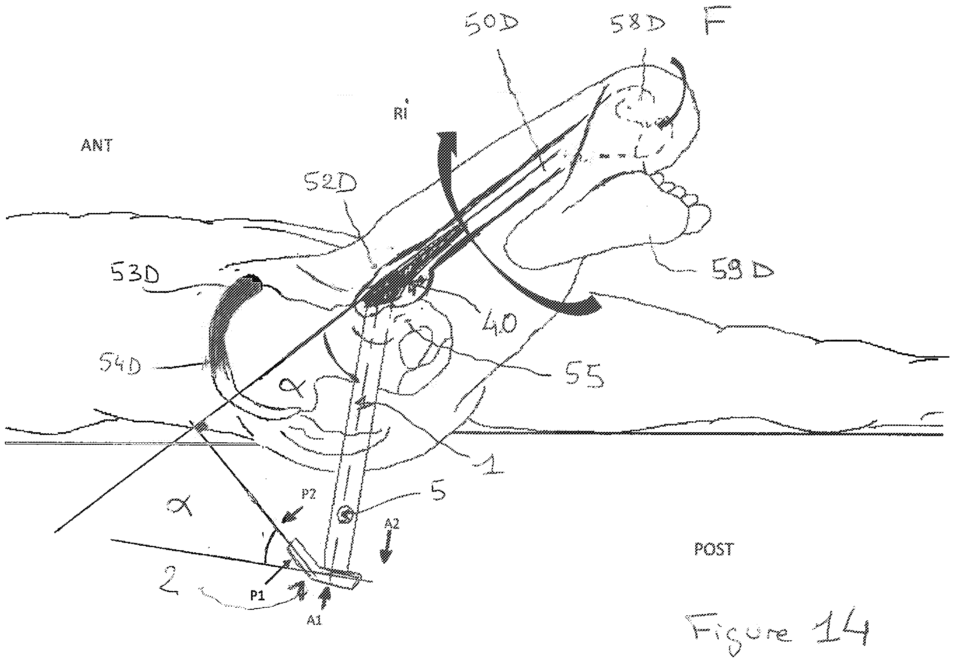

Enfin la figure 14 montre ce porte-outil mis en oeuvre dans une opération par voie postérieure de jambe droite, en position de décubitus latéral.

Sur ces figures 9 à 14 on a porté les références numériques, précédemment utilisées dans les figures 1 à 8. Par ailleurs, on a utilisé les références numériques supplémentaires suivantes : trochanter 52, crête iliaque antérieure 53, crête iliaque 54, cotyle 55, cuisse 56, jambe 57, genou 58 et pied 59. Afin de distinguer les organes respectivement gauche et droite du patient, on a ajouté les suffixes G et D. Sur ces figures on retrouve également les flèches F (flexion), RI (rotation interne) et RE (rotation externe), ainsi que les références POST (postérieur) et ANT (antérieur).

## Revendications

1. Porte-outil chirurgical (10) pour fixer un outil chirurgical (30,40) comportant :
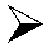 un corps de manche (1) comprenant une partie proximale (6) et une partie
distale (7) délimitées par un décrochement (8),
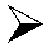 une masse de frappe (2) située à l'extrémité de la partie proximale (6) dudit corps de manche (1),
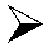 un moyen de fixation (9) d'un outil chirurgical situé à l'extrémité distale (7) du corps de manche (1),
**caractérisé en ce que** ladite masse de frappe (2), de préférence en forme de virgule ou d'accent circonflexe, comprend (i) une partie supérieure (21) et une partie inférieure (24), ladite partie supérieure (21) comprenant une zone de frappe d'insertion axiale fémorale (22) pouvant être de forme légèrement incurvée et s'étendant selon un premier axe longitudinal (B-B') ; et (ii) une zone de frappe d'insertion dans l'axe du manche du porte râpe (23) de forme sensiblement plate s'étendant selon un deuxième axe longitudinal (A-A'), l'angle α formé par l'intersection des premier et deuxième axes longitudinaux (B-B' ; A-A') étant compris entre 20° et 50°, de préférence entre 25° et 45° ; et encore plus préférentiellement environ 35°.

2. Porte-outil selon la revendication 1, **caractérisé en ce que** la partie proximale (6) du corps de manche (1) s'étend de manière longitudinale selon un troisième axe (C-C'), la partie distale (7) dudit corps de manche s'entend de manière longitudinale selon un axe (D-D'), et **en ce que** l'angle β formé par l'intersection des axes (C-C' ; D-D') est compris entre 20° et 45°, de préférence entre 25° et 40°, et encore plus préférentiellement entre 32° et 38°.

3. Porte-outil selon l'une quelconque des revendications 1 à 2, **caractérisé en ce qu'**il comprend un organe de commande (3) dudit moyen de fixation (9), ledit organe de commande (3) comprenant de préférence un actionneur (3a), de préférence logé dans une lumière longitudinale (4), et une tige (3b) logée dans le corps de manche (1) sur laquelle peut agir ledit actionneur (3a).

4. Porte-outil selon l'une quelconque des revendications 1 à 3, **caractérisé en ce que** l'actionneur de l'organe de commande (3) est apte à être déplacé longitudinalement pour verrouiller ou déverrouiller le moyen de fixation (9).

5. Porte-outil selon l'une quelconque des revendications 3 ou 4, **caractérisé en ce que** la tige guide (3b) de l'organe de commande (3) est en appui sur un ressort pour verrouiller ou déverrouiller les moyens de fixation (9).

6. Porte-outil selon la revendication 5, **caractérisé en ce que** la tige guide (3b) présente un décrochement (3c) de même rayon de courbure que le décrochement (8) dudit corps de manche (1).

7. Porte-outil selon l'une quelconque des revendications 1 à 6, **caractérisé en ce qu'**il comprend en outre un orifice d'orientation angulaire (5) situé sur la partie proximale (6) du corps de manche (1) pour l'introduction d'une tige cylindrique afin d'orienter angulairement un outil chirurgical, tel qu'une râpe ou une tige fémorale.

8. Porte-outil selon l'une quelconque des revendications précédentes, **caractérisé en ce que** le moyen de fixation (9) définit un cinquième axe longitudinal (E-E') confondu avec l'axe principal de l'outil chirurgical (30, 40), quand cet outil est fixé sur le porte-outil, ledit cinquième axe (E-E') formant, avec l'axe principal (A1) du corps de manche (1), le même angle (α) que celui formé par l'intersection des premier et deuxième axes longitudinaux (B-B' ; A-A').

9. Porte-outil selon l'une quelconque des revendications précédentes, **caractérisé en ce que** la partie inférieure (24) délimite avec la partie proximale (6) dudit corps de manche (1), en regard de la zone (23) de frappe d'insertion dans l'axe du manche du porte râpe, un espace (27) de passage pour un outil, en vue de l'extraction du porte-outil selon l'axe du manche du porte râpe.

10. Ensemble constitué par un porte-outil selon l'une quelconque des revendications 1 à 9 et un outil chirurgical (30,40), tel qu'une râpe ou une tige fémorale, ledit outil chirurgical étant disposé de manière à ce que son axe longitudinal s'étende selon un cinquième axe (E-E'), l'angle γ formé par l'intersection entre ledit deuxième axe longitudinal (B-B') et ledit cinquième axe (E-E') étant compris entre 87° et 93°, de préférence autour de 90°.

11. Ensemble selon la revendication 10, **caractérisé en ce que** ledit cinquième axe (E-E') forme, avec l'axe principal (A1) du corps de manche (1), le même angle (α) que celui formé par l'intersection des premier et deuxième axes longitudinaux (B-B' ; A-A').

12. Set d'outils chirurgicaux comprenant au moins un porte-outil chirurgical (10) selon l'une quelconque des revendications 1 à 9, et au moins une râpe de finition et/ou au moins une râpe de préparation (40) et éventuellement une tige fémorale (30) de prothèse de hanche aptes à coopérer avec ledit porte-outil chirurgical (10).

## Patentansprüche

1. Chirurgischer Werkzeughalter (10) zum Fixieren eines chirurgischen Werkzeugs (30, 40), umfassend:
- einen Griffkörper (1) mit einem proximalen Abschnitt (6) und einem distalen Abschnitt (7), die durch eine Vertiefung (8) begrenzt sind,
- eine Anschlagmasse (2), die am Ende des proximalen Abschnitts (6) des Griffkörpers (1) angeordnet ist,
- ein Befestigungsmittel (9) für ein chirurgisches Werkzeug, das am distalen Ende (7) angeordnet ist;
**dadurch gekennzeichnet, dass** die Schlagmasse (2), vorzugsweise in Form eines Kommas oder eines Zirkumflex-Akzents, umfasst (i) einen oberen Abschnitt (21) und einen unteren Abschnitt (24), wobei der obere Abschnitt (21) eine axiale Femureinführeinschlagzone (22) aufweist, die leicht gekrümmt sein kann und sich entlang einer ersten Längsachse (B-B ') erstreckt; und (ii) eine Einführzone in die Achse des Griffs des Raspelhalters (23) von im wesentlichen flacher Form, die sich entlang einer zweiten Längsachse (A-A ') erstreckt, wobei der Winkel gebildet wird durch den Schnittpunkt der ersten und zweiten Längsachsen (B-B '; A-A') zwischen 20 ° und 50 °, vorzugsweise zwischen 25 ° und 45 °; und noch bevorzugter ungefähr 35 °.

2. Werkzeughalter nach Anspruch 1, **dadurch gekennzeichnet, dass** sich der proximale Abschnitt (6) des Griffkörpers (1) in Längsrichtung entlang einer dritten Achse (C-C') erstreckt, wobei der distale Abschnitt (7) des Griffkörpers sich in Längsrichtung entlang einer Achse (D - D') erstreckt, und dass der Winkel β, der durch den Schnitt der Achsen (C-C'; D - D') gebildet wird, zwischen 20 ° und 45 ° liegt, vorzugsweise zwischen 25 ° und 40 ° und noch bevorzugter zwischen 32 ° und 38 °.

3. Werkzeughalter nach einem der Ansprüche 1 bis 2, **dadurch gekennzeichnet, dass** er ein Steuerelement (3) des Befestigungsmittels (9) umfasst, wobei das Steuerelement (3) vorzugsweise einen Aktuator (3a) umfasst, der vorzugsweise in einem Längsschlitz (4) untergebracht ist, und eine Stange (3b), die in dem Griffkörper (1) untergebracht ist, auf die der Aktuator (3a) einwirken kann.

4. Werkzeughalter nach einem der Ansprüche 1 bis 3, **dadurch gekennzeichnet, dass** der Aktuator des Steuerorgans (3) in Längsrichtung bewegbar ist, um das Befestigungsmittel (9) zu verriegeln oder zu entriegeln.

5. Werkzeughalter nach einem der Ansprüche 3 oder 4, **dadurch gekennzeichnet, dass** die Führungsstange (3b) des Steuerorgans (3) an einer Feder abgestützt ist, um das Befestigungsmittel (9) zu verriegeln oder zu entriegeln.

6. Werkzeughalter nach Anspruch 5, **dadurch gekennzeichnet, daß** die Führungsstange (3b) eine Ausnehmung (3c) mit gleichem Krümmungsradius wie die Ausnehmung (8) des Griffkörpers (1) aufweist.

7. Werkzeughalter nach einem der Ansprüche 1 bis 6, **dadurch gekennzeichnet, dass** er ferner eine winkelförmige Ausrichtöffnung (5) aufweist, die sich auf dem proximalen Abschnitt (6) des Griffkörpers (1) befindet, zum Einführen eines zylindrischen Stabes, um ein chirurgisches Werkzeug wie eine Raspel oder einen Oberschenkelschaft winkelmäßig auszurichten.

8. Werkzeughalter nach einem der vorhergehenden Ansprüche, **dadurch gekennzeichnet, dass** das Befestigungsmittel (9) eine fünfte Längsachse (E-E') definiert, die mit der Hauptachse des chirurgischen Werkzeugs (30, 40) zusammenfällt, wenn dieses Werkzeug an dem Werkzeughalter befestigt ist, wobei die fünfte Achse (E-E') mit der Hauptachse (A1) des Griffkörpers (1) den gleichen Winkel (α) bildet, wie der, welcher durch den Schnitt der ersten und zweiten Längsachsen (B-B'; A-A') gebildet wird.

9. Werkzeughalter nach einem der vorhergehenden Ansprüche, **dadurch gekennzeichnet, dass** der untere Abschnitt (24) mit dem proximalen Abschnitt (6) des Griffkörpers (1), der der Schlagzone (23) zum Einführen in die Achse des Griffes des Raspelhalters zugewandt ist, einen Durchgang (27) für ein Werkzeug zum Herausziehen des Werkzeughalters entlang der Achse des Griffes des Raspelhalters bildet.

10. Anordnung, gebildet durch einen Werkzeughalternach einem der Ansprüche 1 bis 9 und ein chirurgisches Werkzeug (30, 40), wie eine Raspel oder ein femoraler Schaft, wobei das chirurgische Werkzeug so angeordnet ist, dass seine Längsachse sich entlang einer fünften Achse (E-E ') erstreckt, wobei der Winkel γ, der durch den Schnitt zwischen der zweiten Längsachse (B-B') und der fünften Achse (E-E') gebildet wird, zwischen 87 ° und 93 ° liegt, vorzugsweise um 90 °.

11. Anordnung nach Anspruch 10, **dadurch gekennzeichnet, daß** die fünfte Achse (E-E') mit der Hauptachse (A1) des Griffkörpers (1) den gleichen Winkel (α) bildet wie der Schnitt der ersten und zweiten Längsachsen (B-B '; A-A').

12. Chirurgisches Werkzeugset umfassend mindestens einen chirurgischen Werkzeughalter (10) nach einem der Ansprüche 1 bis 9 und mindestens eine Nachschleifreibe und / oder mindestens eine Präparationsreibe (40) und gegebenenfalls einen Schaft (30) einer Hüftprothese, die geeignet ist, um mit dem chirurgischen Werkzeughalter (10) zusammenzuwirken.

## Claims

1. Surgical tool holder (10) for fixing a surgical tool (30,40) comprising:
- a handle body (1) comprising a proximal portion (6) and a distal portion (7) delimited by a recess (8),
- a striking mass (2) located at the end of the proximal portion (6) of said handle body (1),
- a fixing means (9) of a surgical tool located at the distal portion (7) of the handle body (1),
**characterized in that** said striking mass (2), preferably in the form of a comma or a circumflex accent, comprises (i) an upper portion (21) and a lower portion (24), said upper portion (21) comprising a striking area for axial femoral insertion (22) having a slightly curved shape and extending along a first longitudinal axis (B-B'); and (ii) a striking area for insertion in the axis of the handle of the rasp holder (23) of substantially flat shape extending along a second longitudinal axis (A-A'), the angle α formed by the intersection of the first and second longitudinal axes (B-B'; A-A') being between 20 ° and 50 °, preferably between 25 ° and 45 °, and still more preferably about 35 °.

2. Tool holder according to claim 1, **characterized in that** the proximal portion (6) of the handle body (1) extends longitudinally along a third axis (C-C), the distal portion (7) of said handle body extends longitudinally along an axis (D-D'), and **in that** the angle β formed by the intersection of the axes (C-C; D-D') is between 20 ° and 45 °, preferably between 25 ° and 40 °, and even more preferably between 32 ° and 38 °.

3. Tool holder according to any of claims 1 to 2, **characterized in that** it comprises a control member (3) of said fastening means (9), said control member (3) preferably comprising an actuator (3a), preferably housed in a longitudinal slot (4), and a rod (3b) housed in the handle body (1) on which said actuator (3a) can act.

4. Tool holder according to any of claims 1 to 3, **characterized in that** the actuator of the control member (3) is capable of being moved longitudinally to lock or unlock the fastening means (9).

5. Tool holder according to any of claims 3 or 4, **characterized in that** the guide rod (3b) of the control member (3) is supported on a spring to lock or unlock the fastening means (9).

6. Tool holder according to claim 5, **characterized in that** the guide rod (3b) has a recess (3c) of the same radius of curvature as the recess (8) of said handle body (1).

7. Tool holder according to any one of claims 1 to 6, **characterized in that** it further comprises an angular orientation orifice (5) located on the proximal portion (6) of the handle body (1) for the introducing a cylindrical rod to angularly orient a surgical tool, such as a rasp or a femoral stem.

8. Tool holder according to one of the preceding claims, **characterized in that** the fastening means (9) defines a fifth longitudinal axis (E-E') coinciding with the main axis of the surgical tool (30, 40), when this tool is fixed on the tool holder, said fifth axis (E-E') forming, with the main axis (A1) of the handle body (1), the same angle (α) as that formed by the intersection of the first and second longitudinal axes (B-B'; A-A').

9. Tool holder according to any one of the preceding claims, **characterized in that** the lower portion (24) delimits with the proximal portion (6) of said handle body (1), facing the striking area (23) for insertion in the axis of the handle of the rasp holder, a space (27) passage for a tool, for the extraction of the tool holder along the axis of the handle of the rasp holder.

10. An assembly formed by a tool holder according to any one of claims 1 to 9 and a surgical tool (30,40) such as a rasp or femoral stem, said surgical tool being arranged so that its longitudinal axis extends along a fifth axis (E-E'), the angle γ formed by the intersection between said second longitudinal axis (B-B') and said fifth axis (E-E') being between 87 ° and 93 °, preferably around 90 °.

11. An assembly according to claim 10, **characterized in that** said fifth axis (E-E') forms, with the main axis (A1) of the handle body (1), the same angle (α) as that formed by the intersection of the first and second longitudinal axes (B-B'; A-A').

12. Surgical tool set comprising at least one surgical tool holder (10) according to any one of claims 1 to 9, and at least one finishing rasp and / or at least one preparation rasp (40) and optionally a femoral stem (30) of a hip prosthesis capable of cooperating with said surgical tool holder (10).
